(19) Europäisches Patentamt
European Patent Office
Office européen des brevets

(11) **EP 2 237 877 B1**

(12) **FASCICULE DE BREVET EUROPEEN**

(45) Date de publication et mention
de la délivrance du brevet:
**29.04.2020 Bulletin 2020/18**

(21) Numéro de dépôt: **08865186.4**

(22) Date de dépôt: **16.12.2008**

(51) Int Cl.:
*B01J 20/18* *(2006.01)*    *B01J 20/28* *(2006.01)*
*B01J 20/32* *(2006.01)*    *C07C 7/13* *(2006.01)*
*C07C 15/08* *(2006.01)*    *C07C 17/389* *(2006.01)*
*C07C 29/76* *(2006.01)*    *C07C 37/82* *(2006.01)*
*C07C 201/16* *(2006.01)*

(86) Numéro de dépôt international:
**PCT/FR2008/052317**

(87) Numéro de publication internationale:
**WO 2009/081024 (02.07.2009 Gazette 2009/27)**

(54) **ADSORBANTS ZEOLITIQUES AGGLOMERES, LEUR PROCEDE DE PREPARATION ET LEURS UTILISATIONS**

AGGLOMERIERTE ZEOLITHISCHE ABSORPTIONSMITTEL, HERSTELLUNGSVERFAHREN DAFÜR UND VERWENDUNG DAVON

AGGLOMERATED ZEOLITIC ABSORBENTS, METHOD OF PREPARING THEM AND USES THEREOF

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **20.12.2007 FR 0760098**

(43) Date de publication de la demande:
**13.10.2010 Bulletin 2010/41**

(73) Titulaires:
• **ARKEMA FRANCE**
**92700 Colombes (FR)**
• **IFP Energies nouvelles**
**92500 Rueil-Malmaison (FR)**

(72) Inventeurs:
• **BOUVIER, Ludivine**
**F-64140 Billere (FR)**
• **KIEGER, Stéphane**
**F-78500 Sartrouville (FR)**
• **LAROCHE, Catherine**
**F-69390 Vernaison (FR)**
• **LEFLAIVE, Philibert**
**F-69780 Mions (FR)**
• **FRISING, Tom**
**F-92000 Nanterre (FR)**

(74) Mandataire: **Bandpay & Greuter**
**30, rue Notre-Dame des Victoires**
**75002 Paris (FR)**

(56) Documents cités:
WO-A-00/50166     WO-A-01/24923
WO-A-99/10096     WO-A-2008/009845
FR-A- 2 903 978

**Description**

L'invention concerne des adsorbants zéolitiques agglomérés à base d'un mélange de poudre de zéolite X et de poudre de zéolite LSX, échangées au baryum ou échangées au baryum et au potassium.

Ces adsorbants peuvent être utilisés plus particulièrement pour la production de paraxylène très pur à partir d'une charge d'hydrocarbures aromatiques contenant des isomères à 8 atomes de carbone.

L'utilisation d'adsorbants zéolitiques constitués de zéolites X ou Y échangées avec des ions tels que baryum, potassium ou strontium, seuls ou en mélange, pour adsorber sélectivement le paraxylène dans un mélange d'hydrocarbures aromatiques est bien connu de l'art antérieur.

US 3 558 730, US 3 558 732, US 3 626 020, US 3 663 638 montrent que des adsorbants comprenant des aluminosilicates échangés par du baryum et du potassium ou par du baryum seul (US 3 960 774) sont efficaces pour la séparation du paraxylène d'une coupe en C$_8$ aromatiques.

Un mode de préparation de ces adsorbants est par exemple décrit dans US 3 878 127 et consiste à traiter dans la soude à chaud des agglomérés (zéolite X + liant) de rapport Na$_2$O/Al$_2$O$_3$ strictement inférieur à 0,7 afin de remplacer les cations échangeables de la zéolite (tels que protons ou cations du Groupe IIA par du sodium préalablement à un échange baryum ou baryum+potassium, l'échange préalable au sodium permettant à une plus grande quantité d'ions baryum ou baryum + potassium d'être ajoutés à la structure zéolitique.

Ces adsorbants sont utilisés comme agents d'adsorption dans les procédés en phase liquide, de préférence de type contre-courant simulé similaires à ceux décrits dans US 2 985 589 qui s'appliquent entre autres aux coupes de C$_8$ aromatiques.

Les zéolites que l'on rencontre dans l'art antérieur pour la séparation des xylènes appartiennent au type structural de la faujasite, tout d'abord décrites dans US 2 882 244 et US 3 130 007, qui sont des silico-aluminates cristallisés possédant des cages de taille parfaitement déterminée connectées dans les trois dimensions.

US 6 884 918 préconise une faujasite X de rapport atomique Si/Al entre 1,15 et 1,5. US 6 410 815 enseigne que des adsorbants zéolitiques à base de faujasite à faible teneur en silice, i. e. de rapport atomique Si/Al proche de 1 (que l'on appellera LSX, abréviation de Low Silica X dont la traduction française est zéolite X à faible teneur en silice) sont avantageusement utilisés pour la séparation du paraxylène. WO 01/24923 décrit un procédé d'obtention d'un adsorbant zéolitique aggloméré comprenant un mélange d'au moins une zéolite X de rapport Si/Al égal à 1,25 et d'au moins une zéolite LSX de Si/Al = 1 et dont soit au moins 80% de la somme des sites cationiques échangeables de la totalité des zéolites du mélange est occupé par des cations sodium, ou soit au moins 70% de la somme des sites cationiques échangeables de la totalité des zéolites du mélange est occupé par des cations strontium, le reste des sites échangeables pouvant être occupé par des cations choisis parmi les groupes IA, IIA, IIIA du tableau périodique, les ions trivalents de la série des terres-rares ou lanthanides et un liant inerte.

La zéolite X et la zéolite X à faible teneur en silice présentent donc toutes deux de bonnes performances en terme de sélectivité du paraxylène, mais la synthèse de la zéolite X à faible teneur en silice est plutôt difficile en comparaison avec la synthèse de la zéolite X. En effet, pour abaisser le rapport atomique Si/Al d'une zéolite de type faujasite, il faut augmenter la consommation de soude utilisée dans le procédé de synthèse de la zéolite. Par ailleurs pour cristalliser selon le type structural faujasite lorsque le rapport atomique Si/Al est de 1, il faut ajouter de fortes concentrations de potasse pour inhiber la formation de zéolite A et obtenir uniquement de la zéolite X à faible teneur en silice. Ces fortes consommations de soude et de potasse augmentent le coût de fabrication de ce type de zéolite et posent des problèmes de rejets des effluents.

Dans les références listées ci-dessus, les adsorbants zéolitiques se présentent sous forme de poudre ou sous forme d'agglomérés constitués majoritairement de zéolite et jusqu'à 15% à 20% en poids de liant d'agglomération inerte.

La synthèse des zéolites X et X à faible teneur en silice s'effectuant le plus souvent par nucléation et cristallisation de gels de silico-aluminates, on obtient des poudres dont l'emploi à l'échelle industrielle est particulièrement malaisé (pertes de charges importantes lors des manipulations) et on préfère les formes agglomérées, par exemple sous forme de granulés ou de grains, qui ne présentent pas les inconvénients inhérents aux matières pulvérulentes.

Ces agglomérés, qu'ils soient sous forme de plaquettes, de billes ou d'extrudés, sont en général constitués de zéolite, qui constitue l'élément actif (au sens de l'adsorption) et d'un liant destiné à assurer la cohésion des cristaux de zéolite sous forme d'agglomérés et à conférer aux agglomérés une résistance mécanique suffisante pour résister aux vibrations et aux mouvements auxquels il sont soumis au cours de leurs mises en oeuvre.

La préparation de ces agglomérés s'opère par exemple par empâtage de poudre de zéolite avec une pâte argileuse, dans des proportions de l'ordre de 80 à 90% en poids de poudre de zéolite pour 20% à 10% en poids de liant, puis mise en forme en billes, plaquettes ou extrudés, et traitement thermique à haute température pour cuisson de l'argile et réactivation de la zéolite, l'échange au baryum pouvant être effectué avant et/ou après l'agglomération de la zéolite pulvérulente avec le liant.

On obtient des agglomérés zéolitiques dont la granulométrie est en général de quelques millimètres, et qui, si le choix du liant et la granulation sont faits dans les règles de l'art, présentent un ensemble de propriétés satisfaisantes, en particulier de porosité, de résistance mécanique, de résistance à l'abrasion.

Le brevet US 6 410 815 enseigne que les performances du procédé industriel de séparation du paraxylène dépendent pour une bonne part de l'adsorbant, de sa capacité d'adsorption et de la sélectivité qu'il montre pour le paraxylène dans un milieu constitué de composés aromatiques en $C_8$, typiquement paraxylène, métaxylène, orthoxylène, éthylbenzène, ainsi que d'autre part de l'aptitude des désorbants, tels le toluène et le paradiéthylbenzène, à en désorber le paraxylène adsorbé. La sélectivité $\alpha_{A/B}$ de l'adsorbant pour un composant A par rapport à un composé B est définie comme le rapport des concentrations des composés dans la phase adsorbée divisée par le rapport des concentrations des composés dans la phase non adsorbée à l'équilibre :

$$\alpha_{A/B} = A_{ads}/B_{ads} \text{ x } B_{liq}/A_{liq}$$

où $A_{ads}$ et $B_{ads}$ sont les concentrations du composé A et du composé B dans la phase adsorbée respectivement et $A_{liq}$ et $B_{liq}$ sont les concentrations du composé A et du composé B en phase fluide.

La présente invention a pour objet des adsorbants zéolitiques utilisables notamment pour la séparation du paraxylène d'un mélange de composés aromatiques en $C_8$ présentant d'excellentes performances, notamment en termes de sélectivité et de capacité d'adsorption des xylènes, lesdits adsorbants étant particulièrement adaptés pour une utilisation dans un procédé de séparation du paraxylène en phase liquide, de préférence de type contre-courant simulé.

Les adsorbants zéolitiques agglomérés selon la présente invention comprennent :

- de la poudre de zéolite X échangée à au moins 90% par des ions baryum seuls soit par des ions baryum et des ions potassium, les sites échangeables occupés par le potassium pouvant représenter jusqu'à 1/3 des sites échangeables occupés par les ions baryum+potassium (le complément éventuel étant généralement assuré par des ions alcalins ou alcalino-terreux autres que le baryum et le potassium) ;
- de la poudre de zéolite LSX échangée à au moins 90% par des ions baryum seuls soit par des ions baryum et des ions potassium, les sites échangeables occupés par le potassium pouvant représenter jusqu'à 1/3 des sites échangeables occupés par les ions baryum + potassium (le complément éventuel étant généralement assuré par des ions alcalins ou alcalino-terreux autres que le baryum et le potassium) ;
- et un liant en proportion inférieure ou égale à 20% en poids de la masse totale de l'aggloméré, le rapport molaire de la concentration en zéolite LSX sur la concentration en zéolite X étant compris entre 0,1 et 10.

Sauf indication contraire, dans tout ce qui suit l'expression « compris entre » signifie « compris au sens large ».

Par zéolite X, on entend une zéolite de type X (faujasite) ayant un rapport atomique Si/Al strictement supérieur à 1,15 et inférieur ou égal à 1,5, de manière préférée compris entre 1,2 et 1,4 et de manière avantageuse compris entre 1,2 et 1,3.

Selon un mode de réalisation préféré, la zéolite X des agglomérés selon l'invention est constituée essentiellement de cristaux de diamètre moyen (en nombre) mesuré par MEB et comptage, compris au sens large entre 0,1 et 4 $\mu$m, préférentiellement compris entre 0,1 et 3 $\mu$m, et avantageusement compris entre 0,1 et 2 $\mu$m.

Par zéolite LSX (abréviation de Low Silica X dont la traduction française est zéolite X à faible teneur en silice), on entend ici une zéolite de type X (faujasite) ayant un rapport atomique Si/Al compris entre 1 et 1,15, de préférence égal à 1,00 ± 0,05; les valeurs inférieures à l'unité traduisent les incertitudes analytiques sur la mesure de ce rapport, et les valeurs supérieures, soit la même incertitude analytique, soit un écart tolérable de pureté du produit.

Selon un mode de réalisation préféré, la zéolite LSX des agglomérés selon l'invention est constituée essentiellement de cristaux de diamètre moyen (en nombre) mesuré par MEB et comptage, compris entre 0,1 $\mu$m et 7 $\mu$m, préférentiellement compris entre 0,1 $\mu$m et 4 $\mu$m, avantageusement compris entre 0,1 $\mu$m et 3 $\mu$m et encore plus avantageusement compris entre 0,1 $\mu$m et 2 $\mu$m.

Par liant au sens de la présente invention, on entend une matrice inorganique inerte au sens de l'adsorption comprenant des matériaux amorphes tels que de la silice, des mélanges de silice et d'alumine et/ou des composés tels que des argiles. On ne sortirait pas du cadre de la présente invention si cette matrice contenait des matériaux cristallins zéolitiques autres que de la zéolite X et de la zéolite LSX telles que définies précédemment en quantité ne dépassant pas 5% du poids total de l'aggloméré.

Pour les adsorbants zéolitiques selon l'invention, on définit les deux critères d'efficacité (C.E.) suivants :

$$C.E._{PX/MX} = \alpha_{PX/MX} \text{ x } V_{Dub}$$

et

$$C.E._{PX/EB} = \alpha_{PX/EB} \times V_{Dub}$$

où $\alpha_{PX/MX}$ et $\alpha_{PX/EB}$ représentent les sélectivités entre le paraxylène et métaxylène et entre le paraxylène et l'éthylbenzène respectivement,

et où $V_{Dub}$ est le volume de Dubinin, qui est une estimation du volume microporeux, mesuré selon la méthode décrite ci-après par adsorption d'azote à 77 K.

Le volume de Dubinin est calculé selon la relation de Dubinin-Radushkevich, telle que décrite par LOWELL et al. dans « Characterization of Porous Solids and Powders: Surface Area, Pore Size and Density », chapitre 9, « Micropore Analysis », pages 143-145 :

$$\log V = \log V_0 - D(\log \frac{P}{P_0})^2$$

qui relie le volume V d'azote adsorbé dans le matériau adsorbant à la pression relative $P / P_0$. Le volume de Dubinin est le volume $V_0$, volume maximal de vapeur d'azote que l'on peut condenser dans les micropores du matériau adsorbant. Il s'exprime en $cm^3$ de vapeur d'azote (ramenée aux conditions normales) par gramme d'adsorbant.

Le volume de Dubinin est alors calculé à partir du volume $V_0$ de gaz, qui est ensuite converti en volume de liquide : il s'exprime en $cm^3$ par gramme d'adsorbant, et correspond au volume microporeux disponible pour l'adsorption.

Préalablement à la mesure, l'échantillon est prétraité à 500°C pendant 12 heures sous vide (P < 5.10$^{-6}$ Torr; soit 6,7.10$^{-4}$ Pa). La mesure est ensuite effectuée sur un appareil de type ASAP 2010 M de Micromeritics. Le tracé de l'isotherme est réalisé à l'aide d'une table de pression d'au moins 35 points entre 0,01 et 1 P/P$_0$. On porte sur un diagramme la valeur de log V en fonction de (log(P/P$_0$))$^2$. Le volume de Dubinin est obtenu à partir de l'ordonnée à l'origine de la droite de régression linéaire des points dont (log(P/P$_0$))$^2$ est compris entre 1 et 2 (soit 0,039 < P/P$_0$ < 0,1). L'incertitude de mesure est de ± 0,003.

Des mesures réalisées sur des adsorbants selon l'invention en différentes proportions de zéolite X et de zéolite LSX et avec un taux de liant fixe donné montrent que les deux critères d'efficacité (C.E.) ci-dessus sont significativement supérieurs pour des mélanges présentant des rapports molaires de LSX/X compris entre 0,1 et 10, de manière préférée entre 0,25 et 4,0, et de manière encore préférée entre 0,5 et 2,0 par rapport aux adsorbants ne contenant que de la zéolite X et du liant et aux adsorbants ne contenant que de la zéolite LSX et du liant. Ces rapports sont de préférence compris entre 0,5 et 2,0 et de préférence encore entre 0,54 et 1,86 par rapport aux adsorbants ne contenant que de la zéolite X et du liant et aux adsorbants ne contenant que de la zéolite LSX et du liant.

L'invention concerne également un procédé de préparation des agglomérés selon l'invention qui comprend les étapes suivantes :

- a/ agglomération d'un mélange constitué de poudre de zéolite LSX et de poudre de zéolite X dans un rapport LSX/X variant entre 0,1 et 10 et de manière préférée entre 0,25 et 4, et de manière encore préférée entre 0,5 et 2 avec un liant d'agglomération, de préférence dont au moins une partie du liant contient une ou plusieurs argiles zéolitisables (partie zéolitisable), de préférence au moins 80% en poids, puis mise en forme, séchage et calcination,
- b/ une étape optionnelle de zéolitisation de ladite partie zéolitisable du liant par action d'une solution basique alcaline,
- c/ remplacement d'au moins 90% de l'ensemble des sites échangeables de l'aggloméré par du baryum, suivi du lavage et du séchage du produit ainsi traité (on entend par sites échangeables de l'aggloméré, l'ensemble des sites échangeables de la poudre de zéolite X, de la poudre de zéolite LSX et ceux formés par la zéolitisation éventuelle du liant),
- d/ éventuellement remplacement d'au plus 33% de l'ensemble des sites échangeables de l'aggloméré par du potassium, suivi du lavage et du séchage du produit ainsi traité,
- e/ activation éventuelle du produit obtenu.

De manière préférentielle, le procédé de préparation selon l'invention est constitué essentiellement des étapes a/ à c/ puis e/, ou a/ à e/ définies précédemment.

L'agglomération et la mise en forme (étape a/) peuvent être réalisées selon toutes les techniques connues de l'homme de l'art, telles qu'extrusion, compactage, agglomération.

Le liant d'agglomération a essentiellement pour rôle de faciliter la mise en forme et l'agglomération des poudres de zéolites. De manière préférentielle, ledit liant est inerte au sens de l'adsorption. Le liant d'agglomération mis en oeuvre peut contenir des argiles du type attapulgite, kaolin, sépiolite, bentonite, montmorillonite.

Dans le cas du procédé où l'étape a/ est suivie de l'étape b/ de zéolitisation, le liant d'agglomération contient une partie zéolitisable, i. e. une ou plusieurs argile(s) zéolitisable(s), de préférence de 80% à 100% du poids total de liant. Les argiles zéolitisables appartiennent en général à la famille de la kaolinite, de l'halloysite, des nacrites, des dickites, des kaolins et/ou des métakaolins, auxquelles on peut ajouter une source de silice. Le kaolin est couramment utilisé. La calcination qui suit le séchage est menée à une température en général comprise entre 500 et 600°C.

De plus, lors de l'étape a/, outre la poudre de zéolite et le liant d'agglomération, des additifs peuvent également être mis en œuvre, par exemple des agents porogènes et/ou des additifs destinés à faciliter l'agglomération et/ou à améliorer le durcissement des agglomérés formés.

La poudre de zéolite X mise en œuvre à l'étape a/ peut être issue de la synthèse de cristaux de zéolite X échangée sodium, appelée également zéolite NaX ou 13 X mais on ne sortirait pas du cadre de l'invention en utilisant une poudre ayant subi un ou plusieurs échanges cationiques, entre la synthèse sous forme Na X et sa mise en œuvre à l'étape a/.

La source éventuelle de silice peut être de la silice colloïdale, du silicate, des diatomées, de la perlite, des cendres de calcination (« fly ash » en anglais), du sable et/ou toute autre forme de silice.

La poudre de zéolite LSX mise en œuvre à l'étape a/ peut être issue de la synthèse de cristaux de zéolite sous forme NaKLSX, mais on ne sortirait pas du cadre de l'invention en utilisant une poudre ayant subi un ou plusieurs échanges cationiques, entre la synthèse sous forme NaKLSX et sa mise en œuvre à l'étape a/.

L'étape optionnelle b/ de zéolitisation a notamment pour but d'augmenter la capacité d'adsorption des adsorbants zéolitiques agglomérés. Elle n'est effective que si le liant d'agglomération contient une ou plusieurs argiles zéolitisables.

La zéolitisation peut être pratiquée par immersion du produit issu de l'étape a/ dans une solution basique alcaline, de préférence aqueuse, par exemple une solution aqueuse de soude ou d'un mélange de soude et de potasse, de concentration de préférence supérieure à 0,5 M. Ladite concentration est généralement inférieure à 3 M, de préférence inférieure à 2 M, avantageusement inférieure à 1 M. La zéolitisation s'opère de préférence à chaud (température supérieure à la température ambiante) typiquement à des températures de l'ordre de 80-100°C, afin d'améliorer la cinétique du processus et de réduire les durées d'immersion à moins de 8 heures mais on ne sortirait pas du cadre de l'invention en opérant à des températures plus basses.

Selon ce mode opératoire, on obtient aisément la zéolitisation d'au moins 50%, et de préférence au moins 70 à 82% en poids de la ou des argile(s) zéolitisable(s) contenue(s) dans le liant. On procède ensuite à un lavage à l'eau suivi d'un séchage.

L'étape c/ d'échange au baryum des cations de l'aggloméré s'effectue par mise en contact des agglomérés issus de l'étape optionnelle b/, ou de l'étape a/ ou de l'étape optionnelle d/ avec un sel de baryum, tel que $BaCl_2$, en solution aqueuse à une température comprise entre la température ambiante et 100°C, et de préférence comprise entre 80 et 100°C. Pour obtenir rapidement un taux d'échange en baryum élevé, i. e. supérieur à 90%, on préfère opérer avec un large excès de baryum par rapport aux cations de l'aggloméré que l'on souhaite échanger, typiquement tel que le rapport Ba/Al soit de l'ordre de 5 à 6 en procédant par échanges successifs de façon à atteindre le taux d'échange visé minimum d'au moins 90% et de préférence d'au moins 95%. Dans tout le texte les taux d'échange sont calculés en équivalent et non en molarité.

L'échange éventuel au potassium (étape d/) peut être pratiqué avant et/ou après l'échange au baryum (étape c/) ou de manière simultanée en utilisant une solution contenant les ions baryum et potassium. Il est également possible d'agglomérer un mélange de poudre de zéolite X et de zéolite LSX contenant déjà des ions potassium et s'affranchir (ou non) de l'étape d/.

L'activation (étape e/), dernière étape du procédé d'obtention des adsorbants selon l'invention, a pour but de fixer la teneur en eau, ainsi que la perte au feu de l'adsorbant dans des limites optimales. On procède en général par activation thermique qu'on exécute préférentiellement entre 200 et 300°C pendant un certain temps, typiquement de 1 à 6 heures, en fonction de la teneur en eau et de la perte au feu souhaitées, selon l'utilisation de l'adsorbant qui est visée.

L'invention concerne également les utilisations d'au moins un des adsorbants zéolitiques décrits ci-dessus comme agents d'adsorption susceptibles de remplacer avantageusement les agents d'adsorption décrits dans la littérature à base de zéolite X ou à base de zéolite LSX, échangée au baryum ou échangée au baryum et potassium, et notamment dans les utilisations listées ci-dessous :

* la séparation des isomères aromatiques en $C_8$ et notamment des xylènes,
* la séparation de sucres,
* la séparation d'alcools polyhydriques,
* la séparation d'isomères de toluènes substitués tels que nitrotoluène, diéthyltoluène, toluènediamine,
* la séparation des crésols,
* la séparation des dichlorobenzènes.

L'invention concerne notamment un perfectionnement de procédé de récupération de paraxylène à partir de

coupes d'isomères $C_8$ aromatiques consistant à utiliser comme agent d'adsorption du paraxylène un adsorbant zéolitique selon l'invention mis en oeuvre dans des procédés en phase liquide mais aussi en phase gazeuse.

L'invention concerne particulièrement un procédé de production de paraxylène à haute pureté à partir d'une charge d'hydrocarbures aromatiques contenant des isomères à 8 atomes de carbones comprenant les étapes suivantes :

a) mise en contact, dans des conditions d'adsorption adéquates, de la charge avec un lit d'adsorbant selon l'invention, de manière à adsorber préférentiellement le paraxylène,

b) de mise en contact, dans des conditions de désorption, du lit d'adsorbant avec un désorbant, qui est préférentiellement soit du toluène, soit du paradiéthylbenzène,

c) une étape de soutirage du lit d'adsorbant d'un flux contenant le désorbant et les produits de la charge les moins sélectivement adsorbés,

d) une étape de soutirage du lit d'adsorbant d'un flux contenant le désorbant et le paraxylène,

e) une séparation du flux issu de l'étape c) en un premier flux contenant le désorbant et un second flux contenant les produits de la charge les moins sélectivement adsorbés, et

f) une séparation du flux issu de l'étape d) en un premier flux contenant le désorbant et un second flux contenant du paraxylène à un niveau de pureté supérieure ou égale à 75% et de préférence supérieure ou égale à 99,7%.

Le procédé peut également optionnellement inclure les étapes suivantes :

g) une étape de cristallisation dans un cristalliseur consistant en la cristallisation du paraxylène issu de l'étape f), permettant d'obtenir d'une part des cristaux de paraxylène imbibés de leur liqueur mère, et d'autre part une liqueur mère qui peut être en partie, voir en totalité, recyclée en mélange avec la charge fraîche à l'entrée de l'unité d'adsorption en lit mobile simulé, et

h) une étape de lavage des cristaux issus de l'étape g) à l'issue de laquelle on récupère du paraxylène à une pureté d'au moins 99,7%, et de manière préférée d'au moins 99,8%.

On peut ainsi séparer le produit désiré par chromatographie liquide d'adsorption préparative (en « batch »), avantageusement en lit mobile simulé, c'est-à-dire à contre-courant simulé ou à co-courant simulé, et plus particulièrement à contre-courant simulé.

La séparation chromatographique en lit mobile simulé à contre-courant simulé est bien connue dans l'état de la technique. En règle générale, une unité de séparation en lit mobile simulé comprend au moins une colonne d'adsorption contenant une pluralité de lits d'un adsorbant, interconnectés en boucle fermée. L'unité de séparation en lit mobile simulé comporte au moins trois zones chromatographiques, et éventuellement quatre ou cinq, chacune de ces zones étant constituée par au moins un lit ou une portion de colonne et comprise entre deux points successifs d'alimentation ou soutirage.

Typiquement, on alimente au moins une charge à fractionner et un désorbant (parfois appelé éluant) et l'on soutire au moins un raffinat et un extrait. Les points d'alimentation et de soutirage sont modifiés au cours du temps, typiquement décalés vers le bas d'un lit, et ce, de façon synchrone.

Par définition, on désigne chacune des zones de fonctionnement par un numéro:

- Zone 1 = zone de désorption du produit recherché (contenu dans l'extrait) comprise entre l'injection du désorbant et le prélèvement de l'extrait;
- Zone 2 = zone de désorption des composés du raffinat, comprise entre le prélèvement de l'extrait et l'injection de la charge à fractionner ;
- Zone 3 = zone d'adsorption du produit recherché, comprise entre l'injection de la charge et le soutirage du raffinat , et ;
- Zone 4 située entre le soutirage de raffinat et l'injection du désorbant.

Les conditions opératoires d'une unité industrielle d'adsorption de type contre-courant simulé sont en général les suivantes :

- nombre de lits 6 à 30 ;
- nombre de zones au moins 4 ;
- température 100 à 250°C, de préférence 150 à 190°C ;
- pression comprise entre la pression de bulle des xylènes à la température du procédé et 3 MPa ;
- rapport des débits désorbant sur charge 0,7 à 2,5 (par exemple 0,9 à 1,8 pour une unité d'adsorption seule (stand alone) et 0,7 à 1,4 pour une unité d'adsorption combinée à une unité de cristallisation) ;
- taux de recyclage de 2,5 à 12, de préférence 3,5 à 6.

On pourra se référer à l'enseignement des brevets US 2 985 589, US 5 284 992 et US 5 629 467.

Les conditions opératoires d'une unité industrielle d'adsorption à co-courant simulé sont en général les mêmes que celles fonctionnant à contre-courant simulé à l'exception du taux de recyclage qui est en général compris entre 0,8 et 7. On pourra se référer aux brevets US 4 402 832 et US 4 498 991.

Le solvant de désorption peut être un désorbant dont le point d'ébullition est inférieur à celui de la charge, tel que le toluène mais aussi un désorbant dont le point d'ébullition est supérieur à celui de la charge, tel que le paradiéthylbenzène (PDEB). La sélectivité des adsorbants selon l'invention pour l'adsorption du paraxylène contenu dans des coupes aromatiques en $C_8$ est optimale lorsque leur perte au feu mesurée à 900°C est comprise en général entre 4,0% et 8,0%, de préférence entre 4,7 et 6,7%. De l'eau et un peu de dioxyde de carbone entrent dans la perte au feu.

Une des techniques de choix pour caractériser l'adsorption de molécules en phase liquide sur un solide poreux est de réaliser un perçage. Dans son ouvrage « Principles of Adsorption and Adsorption processes » (« Principes de l'Adsorption et des procédés d'adsorption »), Ruthven définit la technique des courbes de perçage (« breakthrough curves ») comme l'étude de l'injection d'un échelon de constituants adsorbables.

La présente invention est maintenant décrite à l'aide des exemples qui suivent, qui ont pour but d'illustrer certains modes de réalisation de l'invention, sans toutefois limiter la portée de ladite invention, telle qu'elle est revendiquée dans les revendications annexées.

### Exemple 1 (comparatif)

840 g (exprimés en équivalent calciné) de poudre de zéolite X, de rapports atomiques Si/Al = 1,25, et de taille moyenne des cristaux de 1,6 $\mu$m sont agglomérés en les mélangeant intimement avec 160 g de kaolin (exprimés en équivalent calciné) et la quantité d'eau adéquate pour la mise en forme des agglomérés par extrusion. Les extrudés sont séchés, concassés de manière à récupérer des agglomérés dont le diamètre équivalent est égal à 0,7 mm, puis activés à une température de 550°C pendant 2 heures sous courant d'azote.

Ces granulés sont ensuite échangés au moyen d'une solution de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Il est ensuite activé à une température de 200°C pendant 2 heures sous courant d'azote.

Le taux d'échange en baryum est de 97% et la perte au feu (mesurée à 900°C) est de 6,5%.

Le volume microporeux mesuré selon la méthode de Dubinin par adsorption d'azote à 77 K après prétraitement à 500°C pendant 12 heures sous vide est de 0,235 $cm^3$/g.

### Exemple 2 (comparatif)

900 g (exprimés en équivalent calciné) de poudre de zéolite X, de rapport Si/Al = 1,25, et de taille moyenne des cristaux de 1,6 $\mu$m sont agglomérés en les mélangeant intimement avec 170 g de kaolin (exprimés en équivalent calciné), 70 g de silice colloïdale vendue sous la dénomination commerciale Klebosol® 30 (contenant 30% en poids de $SiO_2$ et 0,5% de $Na_2O$) et la quantité d'eau adéquate pour la mise en forme des agglomérés par extrusion. Les extrudés sont séchés, concassés de manière à récupérer des agglomérés dont le diamètre équivalent est égal à 0,7 mm, puis activés à une température de 550°C pendant 2 heures sous courant d'azote.

200 g de granulés ainsi obtenus sont placés dans un réacteur en verre muni d'une double enveloppe régulée à une température de 100 ± 1°C puis on ajoute 1,5 L d'une solution aqueuse de soude de concentration 100 g/L et laisse le milieu réactionnel sous agitation pendant 3 heures. On procède ensuite au lavage des granulés en 3 opérations successives de lavage à l'eau suivi de la vidange du réacteur. On s'assure de l'efficacité du lavage en mesurant le pH final des eaux de lavage qui doit être compris entre 10 et 10,5.

On procède ensuite à un échange baryum dans des conditions opératoires identiques à celles de l'exemple 1 suivi d'un lavage puis d'un séchage à 80°C pendant 2 heures et enfin d'une activation à 200°C pendant 2 heures sous courant d'azote.

Le taux d'échange en baryum est de 95% et la perte au feu (mesurée à 900°C) est de 6,5%.

Le volume microporeux mesuré selon la méthode de Dubinin par adsorption d'azote à 77 K après prétraitement à 500°C pendant 12 heures sous vide est de 0,256 $cm^3$/g.

### Exemple 3 (comparatif)

840 g (exprimés en équivalent calciné) de poudre de zéolite LSX, de rapport Si/Al = 1,02 et de taille moyenne de cristaux de 2,6 $\mu$m sont agglomérés en les mélangeant intimement avec 160 g de kaolin (exprimés en équivalent calciné) et la quantité d'eau adéquate pour la mise en forme des agglomérés par extrusion. Les extrudés sont séchés, concassés de manière à récupérer des agglomérés dont le diamètre équivalent est égal à 0,7 mm, puis calcinés à 550°C sous courant d'azote pendant 2 heures.

L'échange baryum est opéré dans des conditions opératoires identiques à celles de l'exemple 1 à l'exception de la concentration de la solution de $BaCl_2$ qui est de 0,7 M suivi d'un lavage puis d'un séchage à 80°C pendant 2 heures et enfin d'une activation à 200°C pendant 2 heures sous courant d'azote.

Le taux d'échange en baryum est de 98% et la perte au feu (mesurée à 900°C) est de 6,5%. Le volume microporeux mesuré selon la méthode de Dubinin par adsorption d'azote à 77 K après prétraitement à 500°C pendant 12 heures sous vide est de 0,205 $cm^3$/g.

### Exemple 4 (comparatif)

On mélange intimement et agglomère 840 g (exprimés en équivalent calciné) de poudre de zéolite LSX de rapport Si/Al = 1,01, et de taille moyenne de cristaux de 2,6 $\mu$m, 160 g de kaolin (exprimés en équivalent calciné) avec la quantité d'eau adéquate pour opérer par extrusion. Les extrudés sont séchés, concassés de manière à récupérer des agglomérés dont le diamètre équivalent est égal à 0,7 mm, puis calcinés à 550°C sous courant d'azote pendant 2 heures.

200 g de granulés ainsi obtenus sont placés dans un réacteur en verre muni d'une double enveloppe régulée à une température de 95±1°C puis on ajoute 700 mL d'une solution aqueuse de soude de concentration 220 g/L et on laisse le milieu réactionnel sous agitation pendant 3 heures.

On procède ensuite au lavage des granulés en 4 opérations successives de lavage à l'eau suivi de la vidange du réacteur. On s'assure de l'efficacité du lavage en mesurant le pH final des eaux de lavage qui doit être compris entre 10 et 10,5.

On procède ensuite à un échange baryum dans des conditions opératoires identiques à celles de l'exemple 1 suivi d'un lavage puis d'un séchage à 80°C pendant 2 heures et enfin d'une activation à 200°C pendant 2 heures sous courant d'azote.

Le taux d'échange en baryum de cet adsorbant est de 97% et sa perte au feu est de 6,5%. Le volume microporeux mesuré selon la méthode de Dubinin par adsorption d'azote à 77 K après prétraitement à 500°C pendant 12 heures sous vide est de 0,235 $cm^3$/g.

### Exemple 5 (selon l'invention)

On réalise 3 mélanges de poudre constitués de poudre de zéolite X, de rapport Si/Al = 1,25, de taille moyenne des cristaux de 1,6 $\mu$m et de poudre de zéolite LSX, de rapport Si/Al = 1,01 de taille moyenne des cristaux de 2,6 $\mu$m dont les rapports molaires LSX/X sont les suivants : 0,54 ; 1 et 1,86.

900 g (exprimés en équivalent calciné) de chacun de ces mélanges sont ensuite agglomérés en les mélangeant intimement avec 170 g de kaolin (exprimés en équivalent calciné) et la quantité d'eau adéquate pour la mise en forme des agglomérés par extrusion. Les extrudés sont séchés, concassés de manière à récupérer des agglomérés dont le diamètre équivalent est égal à 0,7 mm, puis activés à une température de 550°C pendant 2 heures sous courant d'azote.

L'échange baryum est opéré dans des conditions opératoires identiques à celles de l'exemple 1. Le taux d'échange en baryum de ces 3 produits est donné dans le Tableau 1 ci-dessous. Les volumes microporeux mesurés selon la méthode de Dubinin par adsorption d'azote à 77 K après prétraitement à 500°C pendant 12 heures sous vide sont de 0,229 $cm^3$/g pour l'échantillon de rapport LSX/X = 0,54, 0,236 pour celui de rapport LSX/X = 1 et 0,230 pour celui de rapport LSX/X = 1,86.

-- **Tableau 1** --

|  | LSX/X = 0,54 | LSX/X = 1 | LSX/X = 1,86 |
|---|---|---|---|
| Taux d'échange Baryum (%) | 96 | 97 | 97 |

### Exemple 6

Les 3 échantillons préparés à l'exemple 5 sont activés de façon identique en une seule fois à 200°C pendant 2 heures sous courant d'azote afin de fixer une perte au feu (PAF mesurée à 900°C) de 6,5%.

Ces 3 échantillons ainsi que les échantillons préparés aux exemples 1 et 3 de même perte au feu sont ensuite évalués par un dispositif de chromatographie frontale (technique du perçage) pour déterminer la sélectivité paraxylène-métaxylène ou la sélectivité paraxylène-éthylbenzène respectivement.

La technique du perçage consiste à injecter une charge contenant des quantités égales de paraxylène et de métaxylène ou de paraxylène et d'éthylbenzène ainsi qu'une faible quantité de traceur non adsorbable, sous forme d'échelon, dans une colonne remplie de tamis zéolitique pré-saturé en solvant et à la température du procédé. Ensuite on suit au cours du temps la réponse à cette injection.

Pour cela, on échantillonne l'effluent en sortie de colonne dans des fioles (vials) de faible volume grâce à un collecteur de fractions. Le contenu des vials est ensuite analysé par chromatographie en phase gazeuse. Le résultat obtenu est exprimé sous forme de graphe: composition des différents constituants contenus dans l'effluent en fonction du temps (ou du volume élué). L'exploitation de cette courbe permet de caractériser le comportement thermodynamique et cinétique du tamis. Elle permet de déterminer, entre autres, la sélectivité de séparation entre les constituants A et B $\alpha_{A/B}$ telle que définie précédemment.

Le volume microporeux est également mesuré selon la méthode de Dubinin par adsorption d'azote à 77 K après prétraitement à 500°C pendant 12 heures sous vide. Les volumes de Dubinin ainsi obtenus sont résumés dans le Tableau 2 ; ainsi que les résultats de sélectivités et d'efficacité.

On constate que, de manière surprenante, les critères d'efficacité des échantillons comportant des mélanges de cristaux de X et de cristaux de LSX sont sensiblement plus élevés que ceux des échantillons composés d'une seule des deux structures.

-- **Tableau 2** --

| Tamis | $\alpha_{PX/MX}$[1] | $\alpha_{PX/EB}$[2] | $V_{Dub}$[3] (cm³/g) | C.E.$_{PX/MX}$[4] | C.E.$_{PX/EB}$[5] |
|---|---|---|---|---|---|
| Exemple 1 | 3,3 | 2,2 | 0,235 | 0,776 | 0,517 |
| Exemple 5 LSX/X = 0,54 | 3,65 | 2,55 | 0,229 | 0,836 | 0,584 |
| Exemple 5 LSX/X = 1 | 3,7 | 2,9 | 0,236 | 0,873 | 0,684 |
| Exemple 5 LSX/X = 1,86 | 3,75 | 2,9 | 0,230 | 0,863 | 0,667 |
| Exemple 3 | 4,03 | 2,9 | 0,205 | 0,826 | 0,595 |
| [1] : sélectivité entre le paraxylène et le métaxylène [2] : sélectivité entre le paraxylène et l'éthylbenzène [3] : volume de Dubinin [4] : critère d'efficacité PX/MX [5] : critère d'efficacité PX/EB | | | | | |

**Exemple 7 (selon l'invention)**

On réalise un mélange de rapport molaire LSX/X = 1 de poudre de zéolite X, de rapport Si/Al = 1,25, de taille moyenne des cristaux de 1,6 $\mu$m et de poudre de zéolite LSX, de rapport Si/Al = 1,01 de taille moyenne des cristaux de 2,6 $\mu$m.

900 g (exprimés en équivalent calciné) de chacun de ces mélanges sont ensuite agglomérés en les mélangeant intimement avec 170 g de kaolin (exprimés en équivalent calciné), 70 g de silice colloïdale vendue sous la dénomination commerciale Klebosol® 30 (contenant 30% en poids de $SiO_2$ et 0,5% de $Na_2O$) et la quantité d'eau adéquate pour la mise en forme des agglomérés par extrusion. Les extrudés sont séchés, concassés de manière à récupérer des agglomérés dont le diamètre équivalent est égal à 0,7 mm, puis calcinés à 550°C sous courant d'azote pendant 2 heures.

200 g de granulés ainsi obtenus sont placés dans un réacteur en verre muni d'une double enveloppe régulée à une température de 100 ± 1°C puis on ajoute 1,5 L d'une solution aqueuse de soude de concentration 100 g/L et laisse le milieu réactionnel sous agitation pendant 3 heures. On procède ensuite au lavage des granulés en 4 opérations successives de lavage à l'eau suivi de la vidange du réacteur. On s'assure de l'efficacité du lavage en mesurant le pH final des eaux de lavage qui doit être compris entre 10 et 10,5.

On procède ensuite à un échange baryum dans des conditions opératoires identiques à celles de l'exemple 1 suivi d'un lavage puis d'un séchage à 80°C pendant 2 heures et enfin d'une activation à 200°C pendant 2 heures sous courant d'azote afin de fixer la PAF à 6,5% comme dans l'exemple 5.

Le taux d'échange en baryum est de 97,2%.

Ces échantillons, ont été testés sur le dispositif de chromatographie frontale également décrit dans l'exemple 6 ainsi que deux échantillons comparatifs 100% X et 100% LSX préparés respectivement selon les exemples 2 et 4.

Le volume microporeux est également mesuré selon la méthode de Dubinin par adsorption d'azote à 77 K après prétraitement à 500°C pendant 12 heures sous vide. Les volumes de Dubinin ainsi obtenus sont résumés dans le Tableau 3.

Les résultats de sélectivité et de critères d'efficacités sont réunis dans le Tableau 3, qui reprend également les résultats du Tableau 2 concernant les adsorbants décrits dans l'exemple 6.

-- Tableau 3 --

| Tamis | $\alpha_{PX/MX}$[1] | $\alpha_{PX/EB}$[2] | $V_{Dub}$[3] ($cm^3$/g) | $C.E._{PX/MX}$[4] | $C.E._{PX/EB}$[5] |
|---|---|---|---|---|---|
| Exemple 1 | 3,3 | 2,2 | 0,235 | 0,776 | 0,517 |
| Exemple 2 | 3,33 | 2,25 | 0,256 | 0,852 | 0,576 |
| Exemple 5 LSX/X = 1 | 3,70 | 2,9 | 0,236 | 0,873 | 0,684 |
| Exemple 7 | 3,65 | 2,92 | 0,255 | 0,931 | 0,745 |
| Exemple 3 | 4,03 | 2,9 | 0,205 | 0,826 | 0,595 |
| Exemple 4 | 3,98 | 2,92 | 0,235 | 0,935 | 0,686 |
| [1] : sélectivité entre le paraxylène et le métaxylène<br>[2] : sélectivité entre le paraxylène et l'éthylbenzène<br>[3] : volume de Dubinin<br>[4] : critère d'efficacité PX/MX<br>[5] : critère d'efficacité PX/EB | | | | | |

Les résultats des Tableaux 2 et 3 montrent que, de manière surprenante, les critères d'efficacité sont bien supérieurs avec les agglomérés selon l'invention à base de mélange X/LSX, comparativement avec des tamis exclusivement composés de X ou de LSX.

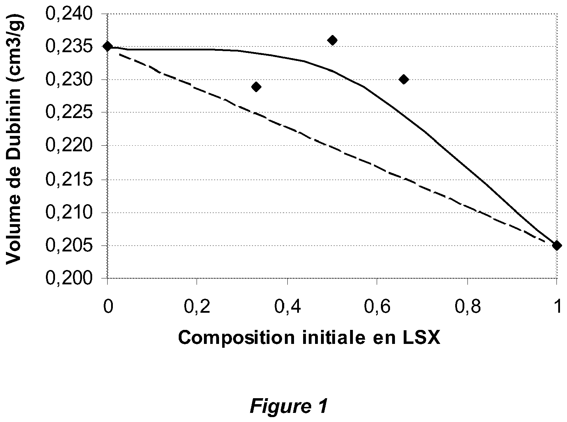

La Figure 1 présente la variation du volume de Dubinin (en ordonnée) en fonction du taux de zéolite LSX présente dans l'aggloméré selon l'invention à base de mélange X/ LSX (en abscisse).

La valeur 0 en abscisse correspond à un aggloméré à base de zéolite X seule, alors que la valeur 1 correspond à un aggloméré à base de zéolite LSX seule.

Le graphique de la Figure 1 montre l'effet inattendu sur le volume de Dubinin, lorsque des agglomérés à base de mélange X/LSX selon l'invention sont obtenus : les volumes de Dubinin observés sont supérieurs à ceux attendus logiquement et qui sont représentés par la ligne droite en pointillés.

## Exemple 8

Afin d'évaluer les performances des tamis en fonction de la PAF, différentes activations de l'échantillon composé uniquement de cristaux de X (préparé selon l'exemple 2) ainsi que celui de rapport molaire LSX/X = 1, préparé selon la méthode décrite dans l'exemple 3, ont été réalisés :

- activation à une température de 230°C pendant 2 heures sous courant d'azote afin de fixer une perte au feu (PAF mesurée à 900°C) de 5% ;
- activation à une température de 170°C pendant 2 heures sous courant d'azote afin de fixer une perte au feu (PAF mesurée à 900°C) de 8%.

Un échantillon composé uniquement de cristaux de LSX (préparé selon l'exemple 4) a été activé à une température de 200°C pendant 2 heures sous courant d'azote afin de fixer une perte au feu (PAF mesurée à 900°C) de 6,5%.

Ces échantillons, ont été testés sur le dispositif de chromatographie frontale également décrit dans l'exemple 6.

Le tableau 4 ci-dessous résume les sélectivités PX/MX et les capacités d'adsorption de xylènes ($cm^3$/g) obtenus pour le tamis exclusivement composé de X ainsi que celui de rapport molaire LSX/X = 1.

-- Tableau 4 --

| | Capacité[1] | $\alpha_{PX/MX}$[2] | $\alpha_{PX/EB}$[3] |
|---|---|---|---|
| | PAF = 5% | | |
| 100%X | 0,225 | 3,5 | 2,5 |
| LSX/X = 1 | 0,229 | 3,7 | 3,12 |

(suite)

| | PAF = 6,5% | | |
|---|---|---|---|
| 100%X | 0,190 | 3,33 | 2,25 |
| LSX/X = 1 | 0,205 | 3,65 | 2,92 |
| 100%LSX | 0,178 | 3,98 | 2,92 |
| | PAF = 8% | | |
| | *Capacité[1]* | $\alpha_{PX/MX}$[2] | $\alpha_{PX/EB}$[3] |
| 100%X | 0,172 | 3,3 | 2,1 |
| LSX/X = 1 | 0,197 | 3,6 | 2,7 |
| [1] : quantité de xylènes adsorbés en $cm^3/g$ <br> [2] : sélectivité entre la paraxylène et le métaxylène <br> [3] : sélectivité entre la paraxylène et l'éthylbenzène | | | |

Pour toutes les PAF testées, les performances des agglomérés à base de mélange X/LSX sont meilleures que les performances des tamis ne contenant que de la zéolite X et que celles des tamis ne contenant que de la zéolite LSX dans des conditions équivalentes.

### Exemple 9

On réalise un mélange de rapport molaire LSX/X = 1 de poudre de zéolite X, de rapport Si/Al = 1,25, de taille moyenne des cristaux de 1,6 $\mu$m et de poudre de zéolite LSX, de rapport Si/Al = 1,01 de taille moyenne des cristaux de 1,6 $\mu$m.

900 g (exprimés en équivalent calciné) de chacun de ces mélanges sont ensuite agglomérés en les mélangeant intimement avec 170 g de kaolin (exprimés en équivalent calciné) et la quantité d'eau adéquate pour la mise en forme des agglomérés par extrusion. Les extrudés sont séchés, concassés de manière à récupérer des agglomérés dont le diamètre équivalent est égal à 0,7 mm, puis calcinés à 550°C sous courant d'azote pendant 2 heures.

200 g de granulés ainsi obtenus sont placés dans un réacteur en verre muni d'une double enveloppe régulée à une température de 100 ± 1°C puis on ajoute 1,5 L d'une solution aqueuse de soude de concentration 100 g/L et laisse le milieu réactionnel sous agitation pendant 3 heures. On procède ensuite au lavage des granulés en 3 opérations successives de lavage à l'eau suivi de la vidange du réacteur. On s'assure de l'efficacité du lavage en mesurant le pH final des eaux de lavage qui doit être compris entre 10 et 10,5.

On procède ensuite à un échange baryum dans des conditions opératoires identiques à celles de l'exemple 1 suivi d'un lavage puis d'un séchage à 80°C pendant 2 heures et enfin d'une activation à 200°C pendant 2 heures sous courant d'azote afin de fixer la PAF à 6,5% comme dans l'exemple 5.

Le taux d'échange en baryum est de 97%.

### Exemple 10 (comparatif)

On réalise un mélange constitué de 50% de granulés préparés selon l'exemple 2 de la présente invention et de 50% de granulés LSX préparés selon l'exemple 4.

On procède ensuite à un échange baryum dans des conditions opératoires identiques à celles de l'exemple 1 suivi d'un lavage puis d'un séchage à 80°C pendant 2 heures et enfin d'une activation à 200°C pendant 2 heures sous courant d'azote afin de fixer la PAF à 6,5% comme dans l'exemple 5.

Le taux d'échange en baryum est de 97,2%.

Ce mélange de granulés de X et de granulés de LSX est ensuite évalué par un dispositif de chromatographie frontale (technique du perçage) pour déterminer la sélectivité paraxylène-métaxylène ou la sélectivité paraxylène-éthylbenzène respectivement.

Les résultats de ces tests sont résumés dans le tableau 5.

-- **Tableau 5** --

| Tamis | $\alpha$ PX/MX[1] | $\alpha$ PX/EB[2] | $V_{Dub}$[3] ($cm^3/g$) | C.E.$_{PX/MX}$[4] | C.E.$_{PX/EB}$[5] |
|---|---|---|---|---|---|

(suite)

| Tamis | $\alpha$ PX/MX[1] | $\alpha$ PX/EB[2] | $V_{Dub}$[3] (cm³/g) | C.E.$_{PX/MX}$[4] | C.E.$_{PX/EB}$[5] |
|---|---|---|---|---|---|
| mélange de granulés X et granulés LSX | 3,3 | 2,2 | 0,240 | 0,790 | 0,527 |

[1] : sélectivité entre le paraxylène et le métaxylène
[2] : sélectivité entre le paraxylène et l'éthylbenzène
[3] : le volume de Dubinin
[4] : le critère d'efficacité PX/MX
[5] : le critère d'efficacité PX/EB

Les résultats du tableau 5 montrent que le simple mélange d'agglomérés de X et d'agglomérés de LSX ne suffit pas pour obtenir la synergie observée pour le mélange de cristaux de X et de LSX à l'intérieur d'un même aggloméré.

**Revendications**

1. Adsorbant zéolitique aggloméré comprenant :

   - de la poudre de zéolite X échangée à au moins 90% par des ions baryum seuls soit par des ions baryum et des ions potassium, les sites échangeables occupés par le potassium pouvant représenter jusqu'à 1/3 des sites échangeables occupés par les ions baryum+potassium ; le complément éventuel étant généralement assuré par des ions alcalins ou alcalino-terreux autres que le baryum et le potassium;
   - de la poudre de zéolite LSX échangée à au moins 90% par des ions baryum seuls soit par des ions baryum et des ions potassium, les sites échangeables occupés par le potassium pouvant représenter jusqu'à 1/3 des sites échangeables occupés par les ions baryum + potassium ; le complément éventuel étant généralement assuré par des ions alcalins ou alcalino-terreux autres que le baryum et le potassium ;
   - et un liant en proportion inférieure ou égale à 20% en poids de la masse totale de l'aggloméré,

   le rapport molaire de la concentration en zéolite LSX sur la concentration en zéolite X étant compris entre 0,1 et 10.

2. Adsorbant selon la revendication 1, **caractérisé en ce que** le rapport molaire de la concentration en zéolite LSX sur la concentration en zéolite X est compris entre 0,25 et 4, et de manière encore préférée entre 0,5 et 2,0.

3. Adsorbant selon la revendication 1, **caractérisé en ce que** le rapport molaire de la concentration en zéolite LSX sur la concentration en zéolite X est compris entre 0,5 et 2,0 et de préférence entre 0,54 et 1,86.

4. Adsorbant selon la revendication 1, **caractérisé en ce que** le la zéolite X est constituée essentiellement de cristaux de diamètre moyen en nombre mesuré par MEB et comptage, compris entre 0,1 et 4 $\mu$m, préférentiellement compris entre 0,1 et 3 $\mu$m, et avantageusement compris entre 0,1 et 2 $\mu$m.

5. Adsorbant selon la revendication 1, **caractérisé en ce que** la zéolite LSX est constituée essentiellement de cristaux de diamètre moyen en nombre mesuré par MEB et comptage, compris entre 0,1 $\mu$m et 7 $\mu$m, préférentiellement compris entre 0,1 $\mu$m et 4 $\mu$m, avantageusement compris entre 0,1 $\mu$m et 3 $\mu$m et encore plus avantageusement compris entre 0,1 $\mu$m et 2 $\mu$m.

6. Procédé de préparation d'un adsorbant selon l'une quelconque des revendications 1 à 5, **caractérisé en ce qu'**il comprend les étapes suivantes :

   - a/ agglomération d'un mélange constitué de poudre de zéolite LSX et de poudre de zéolite X dans un rapport LSX/X variant entre 0,1 et 10 et de manière préférée entre 0,25 et 4,0, et de manière encore préférée entre 0,5 et 2,0 avec un liant d'agglomération, de préférence dont au moins une partie du liant contient une ou plusieurs argiles zéolitisables comme une partie zéolitisable, de préférence au moins 80% en poids, puis mise en forme, séchage et calcination,
   - b/ une étape optionnelle de zéolitisation de ladite partie zéolitisable du liant par action d'une solution basique alcaline,
   - c/ remplacement d'au moins 90% de l'ensemble des sites échangeables de l'aggloméré par du baryum, suivi du lavage et du séchage du produit ainsi traité,

- d/ éventuellement remplacement d'au plus 33% de l'ensemble des sites échangeables de l'aggloméré par du potassium, suivi du lavage et du séchage du produit ainsi traité,
- e/ activation éventuelle du produit obtenu.

**7.** Utilisation d'au moins un adsorbant zéolitique selon l'une quelconque des revendications 1 à 5, comme agent d'adsorption, pour la séparation des isomères aromatiques en $C_8$ et notamment des xylènes, la séparation de sucres, la séparation d'alcools polyhydriques, la séparation d'isomères de toluènes substitués, la séparation des crésols, la séparation des dichlorobenzènes.

**8.** Procédé de séparation des isomères aromatiques en $C_8$ et notamment s des xylènes, de sucres, d'alcools polyhydriques, d'isomères de toluènes substitués, de crésols, ou de dichlorobenzènes, mettant en œuvre au moins un adsorbant selon l'une quelconque des revendications 1 à 5.

**9.** Procédé selon la revendication 8, de récupération de paraxylène à partir de coupes d'isomères C8 aromatiques, par adsorption du paraxylène au moyen d'un adsorbant zéolitique selon l'une quelconque des revendications 1 à 5.

**10.** Procédé selon la revendication 9, qui est mis en œuvre en phase liquide.

**11.** Procédé selon la revendication 9, qui est mis en œuvre en phase gazeuse.

**12.** Procédé de récupération de paraxylène selon l'une quelconque des revendications 9 à 11, **caractérisé en ce qu'**il est de type à lit mobile simulé.

**13.** Procédé de récupération de paraxylène selon l'une quelconque des revendications 9 à 11, **caractérisé en ce qu'**il est de type contre-courant simulé.

**14.** Procédé de récupération de paraxylène selon l'une quelconque des5 revendications 9 à 11 , **caractérisé en ce qu'**il est de type co-courant simulé.

**15.** Procédé de récupération de paraxylène selon l'une quelconque des revendications 9 à 14, dans lequel le désorbant est le toluène ou le paradiéthylbenzène.

**16.** Procédé de récupération de paraxylène selon l'une quelconque des revendications 9 à 15 dans lequel l'adsorbant zéolitique a une perte au feu mesurée à 900°C comprise entre 4,0 et 8%, de préférence entre 4,7 et 6,7%.


**Patentansprüche**

**1.** Agglomeriertes zeolithisches Adsorptionsmittel, umfassend:

- Zeolith X-Pulver, ausgetauscht zu mindestens 90 % durch Bariumionen allein oder durch Bariumionen und Kaliumionen, wobei die austauschbaren Stellen, die durch Kalium besetzt sind, bis 1/3 der austauschbaren Stellen darstellen können, die von der Barium- und Kaliumionen besetzt sind; wobei der eventuelle Zusatz im Allgemeinen von Alkali- oder Erdalkaliionen außer Barium oder Kalium sichergestellt wird.
- Zeolith LSX-Pulver, ausgetauscht zu mindestens 90 % durch Bariumionen allein oder durch Bariumionen und Kaliumionen, wobei die austauschbaren Stellen, die von Kalium besetzt sind, bis zu 1/3 der austauschbaren Stellen darstellen können, die von Barium- und Kaliumionen besetzt sind; wobei der eventuelle Zusatz im Allgemeinen von Alkali- oder Erdalkaliionen außer Barium oder Kalium sichergestellt wird.
- und ein Bindemittel in einer Proportion von weniger als oder gleich 20 Gew.% der Gesamtmasse des Agglomerats, wobei das Molverhältnis der Konzentration von Zeolith LSX zur Konzentration von Zeolith X im Bereich zwischen 0,1 und 10 liegt.

**2.** Adsorptionsmittel nach Anspruch 1, **dadurch gekennzeichnet, dass** das Molverhältnis der Konzentration von Zeolith LSX zur Konzentration von Zeolith X im Bereich zwischen 0,25 und 4, noch bevorzugter zwischen 0,5 und 2,0 liegt.

**3.** Adsorptionsmittel nach Anspruch 1, **dadurch gekennzeichnet, dass** das Molverhältnis der Konzentration von Zeolith LSX zur Konzentration von Zeolith X zwischen 0,5 und 2,0 liegt und vorzugsweise zwischen 0,54 und 1,86.

4. Adsorptionsmittel nach Anspruch 1, **dadurch gekennzeichnet, dass** das Zeolith X im Wesentlichen aus Kristallen mit einem mittleren Durchmesser in einer Anzahl, die durch MEB und Zählung gemessen wird, im Bereich zwischen 0,1 und 4 $\mu$m, vorzugsweise im Bereich zwischen 0,1 und 3 $\mu$m und vorteilhafterweise im Bereich zwischen 0,1 und 2 $\mu$m besteht.

5. Adsorptionsmittel nach Anspruch 1, **dadurch gekennzeichnet, dass** der Zeolith LSX im Wesentlichen aus Kristallen mit einem mittleren Durchmesser in einer Anzahl, die durch MEB und Zählung gemessen wird, im Bereich zwischen 0,1 $\mu$m und 7 $\mu$m, vorzugsweise im Bereich zwischen 0,1 $\mu$m und 4 $\mu$m, vorteilhafterweise im Bereich zwischen 0,1 $\mu$m und 3 $\mu$m und noch vorteilhafter im Bereich zwischen 0,1 $\mu$m und 2 $\mu$m besteht.

6. Verfahren zur Herstellung eines Adsorptionsmittels nach einem der Ansprüche 1 bis 5, **dadurch gekennzeichnet, dass** es die folgenden Schritte umfasst:

- a/ Agglomerieren einer Mischung, die aus Zeolith LSX-Pulver und Zeolith X-Pulver besteht, in einem Verhältnis LSX/X, das zwischen 0,1 und 10 und auf bevorzugte Weise zwischen 0,25 und 4,0 und noch bevorzugter zwischen 0,5 und 2,0 variiert, mit einem Agglomerationsbindemittel, wobei vorzugsweise mindestens ein Teil des Bindemittels ein oder mehrere zeolitisierbare Tone enthält, als einem zeolitisierbaren Teil, vorzugsweise mindestens 80 Gew%, dann Formen, Trocknen und Kalzinieren,
- b/ einen optionalen Schritt des Zeolitisierens des zeolitisierbaren Teils des Bindemittels durch die Wirkung einer basischen Lauge,
- c/ Ersetzen von mindestens 90 % der Gesamtheit der austauschbaren Stellen des Agglomerats durch Barium, gefolgt vom Waschen und Trocknen des so behandelten Produkts
- d/ eventuell Ersetzen von höchstens 33 % der Gesamtheit der austauschbaren Stellen des Agglomerats durch Kalium, gefolgt vom Waschen und Trocknen des so behandelten Produkts
- e/ eventuelles Aktivieren des erhaltenen Produkts.

7. Verwendung mindestens eines zeolithischen Adsorptionsmittels nach einem der Ansprüche 1 bis 5 als Adsorptionsmittel für die Trennung von aromatischen Isomeren in $C_8$ und insbesondere Xylene, die Trennung von Zuckern, die Trennung von mehrwertigen Alkoholen, die Trennung von Isomeren von substituierten Toluenen, die Trennung der Kresole, die Trennung der Dichlorbenzene.

8. Verfahren zur Trennung von aromatischen Isomeren in Cg und insbesondere der Xylene, von Zuckern, mehrwertigen Alkoholen, Isomeren von substituierten Toluenen, Kresolen oder Dichlorbenzenen, wodurch mindestens ein Adsorptioonsmittel nach einem der Ansprüche 1 bis 5 umgesetzt wird.

9. Verfahren nach Anspruch 8, zur Widergewinnung von Paraxylen ausgehend von Schnitten von aromatischen $C_8$-Isomeren durch Adsorption des Paraxylens mit Hilfe eines zeolithischen Adsorptionsmittels nach einem der Ansprüche 1 bis 5.

10. Verfahren nach Anspruch 9, das in der flüssigen Phase umgesetzt wird.

11. Verfahren nach Anspruch 9, das in der gasförmigen Phase umgesetzt wird.

12. Verfahren zur Widergewinnung von Paraxylen nach einem der Ansprüche 9 bis 11, **dadurch gekennzeichnet, dass** es vom Typ mit stimuliertem Wanderbett ist.

13. Verfahren zur Wiedergewinnung von Paraxylen nach einem der Ansprüche 9 bis 11, **dadurch gekennzeichnet, dass** es vom Typ stimulierte Rückströmung ist.

14. Verfahren zur Wiedergewinnung von Paraxylen nach einem der Ansprüche 9 bis 11, **dadurch gekennzeichnet, dass** es vom Typ stimulierter Gleichstrom ist.

15. Verfahren zur Wiedergewinnung von Paraxylen nach einem der Ansprüche 9 bis 14, **dadurch gekennzeichnet, dass** das Desorptionsmittel Toluen oder Paradiethylbenzen ist.

16. Verfahren zur Wiedergewinnung von Paraxylen nach einem der Ansprüche 9 bis 15, wobei das zeolitische Adsorptionsmittel einen Schmelzverlust gemessen bei 900 °C im Bereich zwischen 4,0 und 8 %, vorzugsweise zwischen 4,7 und 6,7 % aufweist.

**Claims**

1. An aggregate zeolitic adsorbent comprising:

   - zeolite X powder exchanged to at least 90% by barium ions alone or by barium ions and potassium ions, the exchangeable sites occupied by potassium possibly representing up to 1/3 of the exchangeable sites occupied by the barium et potassium ions, the possible complement being generally provided by alkali or alkaline-earth ions other than barium and potassium;
   - zeolite LSX powder exchanged to at least 90% by barium ions alone or by barium ions and potassium ions, the exchangeable sites occupied by potassium possibly representing up to 1/3 of the exchangeable sites occupied by the barium plus potassium ions, the possible complement being generally provided by alkali or alkaline-earth ions other than barium and potassium;
   - and a binder in a proportion lower than or equal to 20% by weight of the total mass of the aggregate, the molar ratio of the zeolite LSX concentration to the zeolite X concentration being between 0.1 and 10.

2. The adsorbent according to claim 1, **characterized in that** the molar ratio of the zeolite LSX concentration to the zeolite X concentration is between 0.25 and 4, and even more preferably between 0.5 and 2.0.

3. The adsorbent according to claim 1, **characterized in that** the molar ratio of the zeolite LSX concentration to the zeolite X concentration is between 0.5 and 2.0 and preferably between 0.54 and 1.86.

4. The adsorbent according to claim 1, **characterized in that** the zeolite X essentially consists of crystals having a mean number diameter measured by SEM and counting, of between 0.1 and 4 $\mu$m, preferably between 0.1 and 3 $\mu$m, and advantageously between 0.1 and 2 $\mu$m.

5. The adsorbent according to claim 1, **characterized in that** the zeolite LSX essentially consists of crystals having a mean number diameter measured by SEM and counting, of between 0.1 $\mu$m and 7 $\mu$m, preferably between 0.1 $\mu$m and 4 $\mu$m, and advantageously between 0.1 $\mu$m and 3 $\mu$m and even more advantageously between 0.1 $\mu$m and 2 $\mu$m.

6. Process for preparing an adsorbent according to any one of claims 1 to 5, **characterized in that** it comprises the following steps:

   a) agglomeration of a mixture consisting of zeolite LSX powder and zeolite X powder in a LSX/X ratio varying between 0.1 and 10 and preferably between 0.25 and 4.0, and even more preferably between 0.5 and 2.0 with an agglomeration binder, preferably whereof at least part of the binder contains one or more zeolitizable clays as a zeolitizable portion, preferably at least 80% by weight, followed by shaping, drying and calcination,
   b) an optional step of zeolitization of said zeolitizable portion of the binder by the action of a basic alkaline solution,
   c) replacement of at least 90% of all the exchangeable sites of the aggregate by barium, followed by washing and drying of the product thus treated,
   d) possibly replacement of no more than 33% of all the exchangeable sites of the aggregate by potassium, followed by washing and drying of the product thus treated, and
   e) optional activation of the product obtained.

7. Use of at least one zeolitic adsorbent according to any one of claims 1 to 5, as an adsorption agent, for the separation of C8 aromatic isomers and in particular xylenes, the separation of sugars, the separation of polyhydric alcohols, the separation of isomers of substituted toluenes, the separation of cresols, the separation of dichlorobenzenes.

8. Process for separating C8 aromatic isomers and in particular xylenes, sugars, polyhydric alcohols, isomers of substituted toluenes, cresols, or dichlorobenzenes, using at least one adsorbent according to any one of claims 1 to 5.

9. Process according to claim 8, for recovering paraxylene from C8 aromatic isomer cuts, by adsorption of the paraxylene, using a zeolitic adsorbent according to any one of claims 1 to 5.

**10.** Process according to claim 9, which is carried out in liquid phase.

**11.** Process according to claim 9, which is carried out in gas phase.

**12.** Process for recovering paraxylene according to any one of claims 9 to 11, **characterized in that** it is of the simulated moving bed type.

**13.** Process for recovering paraxylene according to any one of claims 9 to 11, **characterized in that** it is of the simulated counter-current type.

**14.** Process for recovering paraxylene according to any one of claims 9 to 11, **characterized in that** it is of the simulated co-current type.

**15.** Process for recovering paraxylene according to any one of claims 9 to 14, in which the desorbent is toluene or paradiethylbenzene.

**16.** Process for recovering paraxylene, according to any one of claims 9 to 15, in which the zeolitic adsorbent has an ignition loss measured at 900°C of between 4.0 and 8%, preferably between 4.7 and 6.7%.

*Figure 1*

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3558730 A
- US 3558732 A
- US 3626020 A
- US 3663638 A
- US 3960774 A
- US 3878127 A
- US 2985589 A
- US 2882244 A
- US 3130007 A
- US 6884918 B
- US 6410815 B
- WO 0124923 A
- US 5284992 A
- US 5629467 A
- US 4402832 A
- US 4498991 A

**Littérature non-brevet citée dans la description**

- Characterization of Porous Solids and Powders: Surface Area, Pore Size and Density. **LOWELL et al.** Micropore Analysis. 143-145